# FASCICULE DE BREVET EUROPEEN

(11) **EP 1 676 603 B1**
(45) Date de publication et mention de la délivrance du brevet: **03.06.2009**
(21) Numéro de dépôt: 05292432.1
(22) Date de dépôt: 16.11.2005
(51) Int. Cl.: A61Q 5/00, A61K 8/90, A61K 8/87, A61K 8/73, A61K 8/81

(54) **Utilisation de l'association d'un polymère associatif et d'un copolymère amphiphile diblocs pour l'épaississement de compositions cosmétiques**
Verwendung einer Kombination eines assoziativem Polymers mit einem amphiphilen Diblock-Copolymer zum Verdicken von kosmetischen Zubereitungen
Use of an association of an associative polymer and an amphiphilic dibloc copolymer for thickening of cosmetic compositions

(30) Priorité: 22.11.2004 FR 0412382
(43) Date de publication de la demande: 05.07.2006
(60) Demande divisionnaire: 08168141.3
(73) Titulaire: L'ORÉAL, 75008 Paris (FR)
(72) Inventeur: Dubief, Claude, 78150 le Chesnay (FR); Nicolas Morgantini, Luc, 60810 Rully (FR); Bernard, Anne-Laure, 299949 Singapour (SG); Simonet, Frédéric, 77131 Touquin (FR)
(74) Mandataire: Casalonga, Axel

(56) Documents cités:
- EP-A- 1 013 264
- EP-A- 1 426 039
- WO-A-01/16187
- WO-A2-02/45651
- FR-A- 2 679 444
- FR-A- 2 774 899
- FR-A- 2 791 557
- FR-A- 2 817 467
- FR-A- 2 827 514
- FR-A- 2 840 210
- US-A1- 2002 152 556
- US-A1- 2003 219 397
- US-B1- 6 451 299
- "APPLICATION OF ACRYLATES/METHACRYLATES/BEHENETH-25 METHACRYLATE COPOLYMER (ACULYN 28)AS A THICKENER AND SUSPENDING AGENT IN COSMETIC FORMULATIONS AND AS A POLYMERIC EMULSIFIER" RESEARCH DISCLOSURE, KENNETH MASON PUBLICATIONS, WESTBOURNE, GB, vol. 428, décembre 1999 (1999-12), pages 1553-1554,AN428, XP009000073 ISSN: 0374-4353

## Description

La présente invention concerne l'utilisation de l'association d'au moins un polymère associatif comportant au moins une zone hydrophile et au moins une chaîne grasse et d'au moins un copolymère linéaire séquencé diblocs, pour l'épaississement de compositions cosmétiques aqueuses ainsi que des compositions cosmétiques aqueuses épaissies par une telle association.

L'épaississement et/ou la gélification des milieux aqueux par des polymères est depuis longtemps un important sujet de recherche, notamment dans le domaine de la cosmétique et de la pharmacie. L'obtention d'un effet d'épaississement intéressant par un polymère hydrosoluble suppose généralement une masse molaire élevée et un volume hydrodynamique important, et la gélification d'un milieu aqueux nécessite la formation d'un réseau polymère tridimensionnel. L'utilisation de tels polymères de masse molaire très élevée pose cependant un certain nombre de problèmes tels que la texture peu agréable et la difficulté d'étalement des gels obtenus.

Une approche intéressante a consisté à utiliser, en tant qu'épaississants, des polymères capables, en solution, de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis. Les forces d'interactions mises en jeu peuvent être de nature très différente, par exemple de nature électrostatique, de type liaisons hydrogène ou de type interaction hydrophobe.

Des polymères capables de s'associer réversiblement entre eux ou avec d'autres molécules par interaction hydrophobe sont appelés « polymères associatifs ». Il s'agit de polymères comportant une ou plusieurs parties hydrophiles qui les rendent au moins partiellement solubles dans l'eau et une ou plusieurs zones hydrophobes par lesquelles les polymères interagissent et s'associent entre eux ou avec d'autres molécules.

Les polymères associatifs sont couramment utilisés pour épaissir ou gélifier des milieux aqueux. Il est également connu que le pouvoir épaississant de ces polymères associatifs peut être renforcé par l'utilisation conjointe d'agents tensioactifs. Il est généralement admis que, dans ce cas, les chaînes hydrophobes des polymères associatifs participent à la formation de micelles mixtes (agents tensioactifs + zone hydrophobe du polymère associatif) qui constituent les points de jonction d'un réseau polymérique tridimensionnel. L'existence de telles micelles mixtes est généralement mise en évidence par l'existence d'un maximum de la courbe de viscosité en fonction de la concentration d'agent tensioactif pour une concentration de polymère associatif donnée (voir figure unique annexée). La valeur sur l'axe des abscisses du maximum de telles « courbes en cloche » caractérise la stoechiométrie de l'interaction maximale entre les agents tensioactifs et les zones hydrophobes du polymère associatif, et la valeur sur l'axe des ordonnées donne le « facteur d'épaississement maximal» caractérisant la capacité d'épaississement de la combinaison.

Les demandes de brevet EP 1 426 039 et WO 02/45651 décrivent des compositions de teinture capillaire contenant, entre autres, un polymère associatif et des tensioactifs.

Toutefois, tous les agents tensioactifs n'aboutissent pas à un tel effet épaississant renforcé. D'autres posent des problèmes d'innocuité dans des compositions cosmétiques et il peut alors être souhaitable de les remplacer. Enfin, un certain nombre de tels systèmes épaississants à base d'agent tensioactifs et de polymères associatifs sont extrêmement sensibles à la présence de sels et ne permettent par conséquent pas une maîtrise satisfaisante de la viscosité.

La demande de brevet FR 2 827 514 décrit des compositions cosmétiques ou dermatologiques aqueuses, gélifiées au moyen de polymères diblocs de structure A-B, où A est un bloc polymérique hydrosoluble ionique et B un bloc polymérique hydrophobe, dans lesquels la proportion en bloc polymérique ionique A est supérieure ou égale à 60% en poids par rapport au poids total du polymère.

Dans le cadre de ses recherches de nouveaux systèmes épaississants polymériques de milieux aqueux, la demanderesse a constaté que certains copolymères séquencés amphiphiles renforçaient de manière spectaculaire le pouvoir épaississant des polymères associatifs et pouvaient, de ce fait, être utilisés pour remplacer partiellement ou totalement des agents tensioactifs usuels présentant les inconvénients ci-dessus.

L'invention a par conséquent pour objet l'utilisation
- d'au moins un polymère associatif cationique comportant au moins une zone hydrophile et au moins une chaîne grasse en C₈₋₄₀, choisi parmi les celluloses et hydroxyéthylcelluloses quaternisées portant des groupes alkyle, arylalkyle, alkylaryle en C₈₋₃₀, et
- d'au moins un copolymère linéaire séquencé diblocs anionique constitué d'un bloc hydrophobe et d'un bloc hydrophile, le rapport en poids du bloc hydrophobe au bloc hydrophile du copolymère diblocs étant compris entre 1/20 et 20/1, à l'exclusion des copolymères séquencés d'oxyde d'éthylène et d'oxyde de propylène, des copolymères séquencés à motifs uréthane et des copolymères séquencés à motifs siloxane,
pour l'épaississement ou la gélification de compositions cosmétiques aqueuses.

Elle a également pour objet des compositions cosmétiques contenant, dans un milieu aqueux physiologiquement acceptable, l'association
- d'au moins un polymère associatif cationique comportant au moins une zone hydrophile et au moins une chaîne grasse en C₈₋₄₀, choisi parmi les celluloses et hydroxyéthylcelluloses quaternisées portant des groupes alkyle, arylalkyle, alkylaryle en C₈₋₃₀,
- d'au moins un copolymère linéaire séquencé diblocs anionique constitué d'un bloc hydrophobe et d'un bloc hydrophile, le rapport en poids du bloc hydrophobe au bloc hydrophile du copolymère diblocs étant compris entre 1/20 et 20/1, à l'exclusion des copolymères séquencés d'oxyde d'éthylène et d'oxyde de propylène, des copolymères séquencés à motifs uréthane et des copolymères séquencés à motifs siloxane.

On entend par « milieu aqueux » au sens de la présente invention un milieu solvant, liquide à température ambiante et à pression atmosphérique, contenant une importante fraction d'eau, de préférence au moins égale à 30 % en poids, en particulier au moins égale à 50 % en poids et idéalement supérieure à 75 % en poids, rapporté au poids total du milieu solvant, la fraction complémentaire étant constituée de solvants organiques physiologiquement acceptables miscibles à l'eau, tels que les alcools inférieurs en C₁-C₄, en particulier l'éthanol, l'isopropanol, le tertio-butanol et le n-butanol, ou les alkylèneglycols comme le propylèneglycol et le glycérol.

De manière analogue, des « compositions aqueuses » au sens de la présente invention sont des compositions contenant un milieu aqueux tel que défini ci-dessus.

Les copolymères linéaires séquencés diblocs, utilisables dans la présente invention, sont des copolymères dits "amphiphiles", à savoir des copolymères comportant à la fois un bloc hydrophobe et un bloc hydrophile.

On entend par "bloc hydrophobe" selon la présente invention un bloc comprenant au moins 75 % en moles de monomères insolubles dans l'eau et par "bloc hydrophile", un bloc comprenant au moins 75 % en moles de monomères hydrosolubles.

Les monomères hydrosolubles formant le bloc hydrophile des copolymères diblocs utilisés dans la présente invention peuvent être utilisés seuls ou sous forme de mélange contenant deux ou plusieurs monomères différents.

La demanderesse a obtenu des résultats particulièrement intéressants avec des copolymères séquencés diblocs anioniques, comportant un bloc hydrophile formé de monomères hydrosolubles anioniques ou d'un mélange de monomères hydrosolubles anioniques et non-ioniques.

On entend au sens de la présente invention par "monomères ou polymères solubles" dans un milieu donné, des monomères ou polymères qui, introduits dans ledit milieu à 25°C, à une concentration en poids égale à 0,5 %, si besoin est, neutralisés, permettent l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide ou l'anhydride maléique, l'acide 2-acrylamido-2-méthylpropanesulfonique, l'acide styrène-sulfonique, l'acide vinylsulfonique et l'acide vinylphosphonique.

Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Les monomères insolubles dans l'eau formant le bloc hydrophobe des copolymères diblocs sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁₋₁₀, d'aryle en C₆₋₁₀ ou d'aralkyle en C₆₋₁₀ et les méthacrylates d'alkyle en C₁₋₁₀, d'aryle en C₆₋₁₀ ou d'aralkyle en C₆₋₁₀, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Comme indiqué ci-dessus à propos de la définition des blocs hydrophobe et hydrophile des copolymères diblocs, les monomères insolubles dans l'eau et les monomères hydrosolubles représentent au moins 75 % en moles respectivement des blocs hydrophobe et hydrophile. Autrement dit, le bloc hydrophobe peut comprendre jusqu'à 25 % en moles d'un ou de plusieurs monomères hydrosolubles. Cette proportion est de préférence au plus égale 10 % en moles et, idéalement, inférieure ou égale à 5 % en moles.

De manière analogue, le bloc hydrophile peut comprendre jusqu'à 25 % en moles, de préférence jusqu'à 10 % en moles, et idéalement jusqu'à 5 % en moles, d'un ou de plusieurs monomères insolubles dans l'eau.

Les copolymères linéaires diblocs utilisés englobent bien entendu également ceux dans lesquels le bloc hydrophile et le bloc hydrophobe sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.

Le rapport en poids du bloc hydrophobe au bloc hydrophile du copolymère séquencé est compris entre 1/20 et 20/1, et en particulier entre 1/10 et 10/1.

Les copolymères séquencés diblocs, pour pouvoir renforcer efficacement le pouvoir épaississant des polymères associatifs décrits plus en détail ci-après, doivent être au moins partiellement solubles et dissous dans le milieu aqueux utilisé.

Dans un mode de réalisation préféré de l'invention, les copolymères séquencés diblocs de la présente invention comportent une séquence homopolymère hydrophobe et une séquence homopolymère hydrophile anionique.

On peut citer à titre d'exemples de tels polymères préférés comportant deux séquences homopolymères, les copolymères polystyrène-poly(acrylate de sodium) et polystyrène-poly(styrène sulfonate de sodium).

Avantageusement, la viscosité des compositions de l'invention est comprise entre 100 m.Pa.s et 10⁶ m.Pa.s à la température de 25°C et à un taux de cisaillement de 1s⁻¹. Elle peut être mesurée à l'aide d'un appareil du type HAAKE RS 600.

La quantité de copolymères séquencés diblocs utilisée dans la présente invention dépend bien entendu de la nature et de la quantité de polymère associatif utilisée, du facteur d'épaississement du couple copolymère diblocs-polymère associatif, et surtout de la viscosité que l'on souhaite obtenir. Les compositions cosmétiques de la présente invention contiennent des concentrations de copolymères séquencés diblocs comprises entre 0,001 et 20%, et de préférence entre 0,005 et 10%, et encore plus préférentiellement entre 0,01 et 5%, rapporté au poids total de la composition.

La concentration en poids des polymères associatifs dans la composition selon l'invention peut varier d'environ 0,01 à 20 % du poids total de la composition. Plus préférentiellement, cette concentration est comprise entre 0,05 et 10%, et encore plus préférentiellement entre 0,1 et 5% par rapport au poids total de la composition.

Avantageusement, le rapport pondéral polymère(s) associatif(s)/ polymère(s) diblocs est compris entre 0,01 et 100. De préférence, ce rapport est compris entre 1 et 50, et encore plus préférentiellement entre 5 et 30.

Les polymères associatifs cationiques utilisés dans la présente invention sont les dérivés à chaîne grasse de celluloses ou hydroxyéthylcelluloses quaternisées choisis parmi les suivants :
- les celluloses quaternisées modifiées par des groupements comportant au moins une chaîne grasse, tels que les groupes alkyle, arylalkyle, alkylaryle comportant de 8 à 30 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant de 8 à 30 atomes de carbone, ou des mélanges de ceux-ci.

Les radicaux aryle désignent de préférence les groupements phényle, benzyle, naphtyle ou anthryle.

On peut indiquer comme exemples d'alkylhydroxyéthyl celluloses quaternisées à chaînes grasses en C₈-C₃₀, les produits QUATRISOFT LM 200^{®}, QUATRISOFT LM-X 529-18-A^{®}, QUATRISOFT LM-X 529-18B^{®} (alkyle en C₁₂) et QUATRISOFT LM-X 529-8^{®} (alkyle en C₁₈) commercialisés par la société AMERCHOL et les produits CRODACEL QM^{®}, CRODACEL QL^{®} (alkyle en C₁₂) et CRODACEL QS^{®} (alkyle en C₁₈) commercialisés par la société CRODA.

Avantageusement, les polymères associatifs utilisés selon l'invention possèdent au moins deux chaînes grasses en C₈-C₄₀.

Les compositions cosmétiques aqueuses de la présente invention épaissies par l'association d'au moins un polymère associatif cationique et d'au moins un copolymère diblocs amphiphile anionique peuvent être des compositions de lavage des matières kératiniques telles que des savons liquides et shampooings, des compositions d'après-shampooing, des compositions de teinture directe ou d'oxydation des cheveux, des compositions réductrices pour la déformation permanente des cheveux ou pour le décapage de la couleur, des compositions oxydantes pour la coloration ou la décoloration des cheveux, des compositions alcalines pour le défrisage, des compositions de mise en forme temporaire des cheveux.

Ces compositions peuvent être aussi des compositions pour le traitement de la peau et des muqueuses, telles que des compositions anti-solaires, des compositions pour le maquillage des lèvres et/ou de la peau.

Elles peuvent être aussi des compositions de maquillage des cils, telles que des mascaras.

Préférentiellement, les compositions de l'invention sont des compositions capillaires.

Elles peuvent contenir par conséquent des principes actifs cosmétiques ou des additifs de formulation couramment utilisés, tels que des polymères filmogènes anioniques, non-ioniques, cationiques ou amphotères, des épaississants polymériques naturels ou synthétiques différents des polymères associatifs et des copolymères diblocs amphiphiles décrits ci-dessus, des épaississants non-polymériques comme des acides ou des électrolytes, des agents tensioactifs non-ioniques, anioniques, cationiques ou zwitterioniques, des agents nacrants, des agents opacifiants, des colorants ou pigments, des parfums, des huiles minérales, végétales et/ou synthétiques, des cires dont les céramides, des vitamines, des filtres UV, des agents antipelliculaires, des agents anti-radicalaires, des agents plastifiants, des agents conservateurs ou des agents de stabilisation du pH.

L'homme du métier veillera à choisir les éventuels additifs et leur quantité de manière à ce qu'ils ne nuisent pas aux propriétés intéressantes des compositions de lavage des fibres kératiniques de la présente invention, à savoir l'effet épaississant de l'association de polymères revendiquée.

## Revendications

1. Composition cosmétique contenant, dans un milieu aqueux physiologiquement acceptable, l'association
• d'au moins un polymère associatif cationique comportant au moins une zone hydrophile et au moins une chaîne grasse en C₈₋₄₀ choisi parmi, les celluloses et hydroxyéthylcelluloses quaternisées portant des groupes alkyle, arylalkyle, alkylaryle en C₈₋₃₀, et
• d'au moins un copolymère linéaire séquence diblocs, anionique, constitué d'un bloc hydrophobe et d'un bloc hydrophile le rapport en poids du bloc hydrophobe au bloc hydrophile du copolymère dibloc étant compris entre 1/20 et 20/1, à l'exclusion des copolymères séquencés d'oxyde d'éthylène et d'oxyde de propylène, des copolymères séquencés à motifs uréthanne et des copolymères séquencés à motifs siloxane.

2. Composition cosmétique selon la revendication 1, **caractérisée par le fait que** le polymère associatif comportant au moins une zone hydrophile et au moins une chaîne grasse en C₈₋₄₀ est soluble dans le milieu aqueux utilisé.

3. Composition cosmétique selon l'une quelconque des revendications précédentes, **caractérisée par le fait que** le ou les copolymères linéaires séquencés diblocs sont solubles dans le milieu aqueux utilisé.

4. Composition cosmétique selon l'une quelconque des revendications précédentes, **caractérisée par le fait que** le bloc hydrophile du copolymère linéaire séquencé diblocs est formé de monomères hydrosolubles anioniques ou d'un mélange de monomères hydrosolubles anioniques et non-ioniques.

5. Composition cosmétique selon la revendication 4, **caractérisée par le fait que** les monomères hydrosolubles anioniques sont choisis parmi les acides carboxyliques à insaturation éthylénique, l'acide 2-acrylamido-2-méthylpropanesulfonique, l'acide styrène sulfonique, l'acide vinylsulfonique et l'acide vinylphosphonique, et les sels de ces acides.

6. Composition cosmétique selon la revendication 4, **caractérisée par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

7. Composition cosmétique selon l'une quelconque des revendications précédentes, **caractérisée par le fait que** le bloc hydrophobe du copolymère linéaire séquencé diblocs est formé de monomères insolubles dans l'eau, choisis parmi les monomères vinylaromatiques, les diènes et les dérivés alkylés des diènes, le chloroprène, les acrylates d'alkyle en C₁₋₁₀, d'aryle en C₆₋₁₀ ou d'aralkyle en C₁₋₁₀ les méthacrylates d'alkyle en C₁₋₁₀, d'aryle en C₆₋₁₀ ou d'aralkyle en C₁₋₁₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

8. Composition cosmétique selon l'une quelconque des revendications précédentes, **caractérisée par le fait que** le bloc hydrophile du copolymère linéaire séquencé diblocs contient jusqu'à 25 % en moles, dé, préférence jusqu'à 10 % en moles, et idéalement jusqu'à 5 % en moles, d'un ou de plusieurs monomères insolubles dans l'eau selon la revendication 7.

9. Composition cosmétique selon l'une quelconque des revendications précédentes, **caractérisée par le fait que** le bloc hydrophobe du copolymère linéaire séquence diblocs contient jusqu'à 25 % en moles, de préférence jusqu'à 10 % en moles, et idéalement jusqu'à 5 % en moles, d'un ou de plusieurs monomères hydrosolubles selon l'une des revendications 5 et 6.

10. Composition cosmétique selon l'une quelconque des revendications précédentes, **caractérisée par le fait que** le ou les copolymères linéaires séquences diblocs comportent une séquence homopolymère hydrophobe et une séquence homopolymère hydrophile anionique.

11. Composition cosmétique selon la revendication 10, **caractérisée par le fait que** la séquence homopolymère hydrophobe est du polystyrène et la séquence homopolymère hydrophile anionique est du poly(acrylate de sodium) ou du poly(styrènesulfonate de sodium).

12. Composition cosmétique selon l'une quelconque des revendications précédentes, **caractérisée par le fait que** la concentration en polymère associatif est comprise entre 0,01 et 20%, de préférence entre 0,05 et 10%, et plus préférentiellement entre 0,1 et 5% du poids total de la composition.

13. Composition cosmétique selon l'une quelconque des revendications précédentes, **caractérisée par le fait que** la concentration en copolymère linéaire séquence diblocs est comprise entre 0,001 et 20%, de préférence entre 0,005 et 10%, et plus préférentiellement entre 0,01 et 5% du poids total de la composition.

14. Composition cosmétique selon l'une quelconque des revendications précédentes, **caractérisée par le fait que** le rapport pondéral polymères(s) associatif(s)/polymère(s) diblocs est compris entre 0,01 et 100, de préférence entre 1 et 50, et plus préférentiellement entre 5 et 30.

15. Utilisation dans le domaine capillaire de l'association
• d'au moins un polymère associatif cationique comportant au moins une zone hydrophile et au moins une chaîne grasse en C₈₋₄₀ choisi parmi, les celluloses et hydroxyéthylcelluloses quaternisées portant des groupes alkyle, arylalkyle, alkylaryle en C₈₋₃₀, et
• d'au moins un copolymère linéaire séquence diblocs anionique, constitué d'un bloc hydrophobe et d'un bloc hydrophile le rapport en poids du bloc hydrophobe au bloc hydrophile du copolymère dibloc étant compris entre 1/20 et 20/1, à l'exclusion des copolymères séquencés d'oxyde d'éthylène et d'oxyde de propylène, des copolymères séquences à motifs uréthane et des copolymères séquences à motifs siloxane.

16. Utilisation de l'association
• d'au moins un polymère associatif cationique comportant au moins une zone hydrophile et au moins une chaîne grasse en C₈₋₄₀ choisi parmi, les celluloses et hydroxyéthylcelluloses quaternisées portant des groupes alkyle, arylalkyle, alkylaryle en C₈₋₃₀, et
• d'au moins un copolymère linéaire séquencé diblocs, anionique, constitué d'un bloc hydrophobe et d'un bloc hydrophile le rapport en poids du bloc hydrophobe au bloc hydrophile du copolymère dibloc étant compris entre 1/20 et 20/1, à l'exclusion des copolymères séquencés d'oxyde d'ethylène et d'oxyde de propylène, des copolymères séquencés à motifs uréthanne et des copolymères séquencés à motifs siloxane,
pour l'épaississement ou la gélification de compositions cosmétiques aqueuses.

17. Utilisation de l'association selon la revendication 16 pour l'épaississement ou la gélification de compositions capillaires aqueuses.

## Claims

1. Cosmetic composition comprising, in a physiologically acceptable aqueous medium, a combination:
• of at least one cationic associative polymer containing at least one hydrophilic part and at least one C₈₋₄₀ fatty chain and selected from quaternized celluloses and hydroxyethylcelluloses which carry C₈₋₃₀ alkyl, arylalkyl and/or alkylaryl groups, and
• of at least one anionic linear diblock block copolymer composed of a hydrophobic block and a hydrophilic block, the ratio by weight of the hydrophobic block to the hydrophilic block of the diblock copolymer being between 1/20 and 20/1, with the exception of block copolymers of ethylene oxide and propylene oxide, block copolymers with urethane units and block copolymers with siloxane units.

2. Cosmetic composition according to Claim 1, **characterized in that** the associative polymer containing at least one hydrophilic part and at least one C₈₋₄₀ fatty chain is soluble in the aqueous medium used.

3. Cosmetic composition according to either of the preceding claims, **characterized in that** the linear diblock block copolymer or copolymers are soluble in the aqueous medium used.

4. Cosmetic composition according to any one of the preceding claims, **characterized in that** the hydrophilic block of the linear diblock block copolymer is formed from anionic water-soluble monomers or from a mixture of anionic and nonionic water-soluble monomers.

5. Cosmetic composition according to Claim 4, **characterized in that** the anionic water-soluble monomers are selected from ethylenically unsaturated carboxylic acids, 2-acrylamido-2-methylpropanesulphonic acid, styrenesulphonic acid, vinylsulphonic acid and vinylphosphonic acid, and the salts of these acids.

6. Cosmetic composition according to Claim 4, **characterized in that** the nonionic water-soluble monomers are selected from acrylamide, N-C₁₋₆ alkyl-acrylamides or N,N-di-C₁₋₃ alkyl-acrylamides, polyethylene glycol acrylate, polyethylene glycol methacrylate, N-vinylacetamide, N-methyl-N-vinylacetamide, N-vinylformamide, N-methyl-N-vinylformamide, N-vinyl lactams containing a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

7. Cosmetic composition according to any one of the preceding claims, **characterized in that** the hydrophobic block of the linear diblock block copolymer is formed from water-insoluble monomers selected from vinylaromatic monomers, dienes and alkyl derivatives of dienes, chloroprene, C₁₋₁₀ alkyl acrylates, C₆₋₁₀ aryl acrylates or C₁₋₁₀ aralkyl acrylates, C₁₋₁₀ alkyl methacrylates, C₆₋₁₀ aryl methacrylates or C₁₋₁₀ aralkyl methacrylates, vinyl acetate, vinyl ethers of formula CH₂=CH-O-R and allyl ethers of formula CH₂=CH-CH₂-O-R in which R represents a C₁₋₆ alkyl group, acrylonitrile, vinyl chloride, vinylidene chloride, caprolactone, ethylene, propylene and fluorinated vinyl monomers or vinyl monomers containing a perfluorinated chain.

8. Cosmetic composition according to any one of the preceding claims, **characterized in that** the hydrophilic block of the linear diblock block copolymer contains up to 25 mol%, preferably up to 10 mol%, and ideally up to 5 mol% of one or more water-insoluble monomers according to Claim 7.

9. Cosmetic composition according to any one of the preceding claims, **characterized in that** the hydrophobic block of the linear diblock block copolymer contains up to 25 mol%, preferably up to 10 mol%, and ideally up to 5 mol% of one or more water-soluble monomers according to either of Claims 5 and 6.

10. Cosmetic composition according to any one of the preceding claims, **characterized in that** the linear diblock block copolymer or copolymers contain a hydrophobic homopolymer block and an anionic hydrophilic homopolymer block.

11. Cosmetic composition according to Claim 10, **characterized in that** the hydrophobic homopolymer block is polystyrene and the anionic hydrophilic homopolymer block is poly(sodium acrylate) or poly(sodium styrenesulphonate).

12. Cosmetic composition according to any one of the preceding claims, **characterized in that** the concentration of associative polymer is between 0.01% and 20%, preferably between 0.05% and 10%, and more preferably between 0.1% and 5% of the total weight of the composition.

13. Cosmetic composition according to any one of the preceding claims, **characterized in that** the concentration of linear diblock block copolymer is between 0.001% and 20%, preferably between 0.005% and 10%, and more preferably between 0.01% and 5% of the total weight of the composition.

14. Cosmetic composition according to any one of the preceding claims, **characterized in that** the associative polymer(s)/diblock polymer(s) weight ratio is between 0.01 and 100, preferably between 1 and 50, and more preferably between 5 and 30.

15. Use in the hair treatment field of a combination
• of at least one cationic associative polymer containing at least one hydrophilic part and at least one C₈₋₄₀ fatty chain and selected from quaternized celluloses and hydroxyethylcelluloses which carry C₈₋₃₀ alkyl, arylalkyl and/or alkylaryl groups, and
• of at least one anionic linear diblock block copolymer composed of a hydrophobic block and a hydrophilic block, the ratio by weight of the hydrophobic block to the hydrophilic block of the diblock copolymer being between 1/20 and 20/1, with the exception of block copolymers of ethylene oxide and propylene oxide, block copolymers with urethane units and block copolymers with siloxane units.

16. Use of a combination
• of at least one cationic associative polymer containing at least one hydrophilic part and at least one C₈₋₄₀ fatty chain and selected from quaternized celluloses and hydroxyethylcelluloses which carry C₈₋₃₀ alkyl, arylalkyl and/or alkylaryl groups, and
• of at least one anionic linear diblock block copolymer composed of a hydrophobic block and a hydrophilic block, the ratio by weight of the hydrophobic block to the hydrophilic block of the diblock copolymer being between 1/20 and 20/1, with the exception of block copolymers of ethylene oxide and propylene oxide, block copolymers with urethane units and block copolymers with siloxane units, for thickening or gelling aqueous cosmetic compositions.

17. Use of the combination according to Claim 16 for thickening or gelling aqueous hair treatment compositions.

## Patentansprüche

1. Kosmetische Zusammensetzung, enthaltend in einem physiologisch akzeptablen wässrigen Medium die Kombination aus:
- mindestens einem assoziativen kationischen Polymer, das mindestens einen hydrophilen Teil und mindestens eine C₈₋₄₀-Fettkette aufweist und das unter den Cellulosen und Hydroxyethylcellulosen ausgewählt ist, die quaternisiert sind und Alkyl-, Arylalkyl-, Alkylarylgruppen mit 8 bis 30 Kohlenstoffatomen tragen, und
- mindestens einem anionischen linearen Zweiblock-Sequenzcopolymer, das aus einem hydrophoben Block und einem hydrophilen Block besteht, wobei das Gewichtsverhältnis des hydrophoben Blocks zu dem hydrophilen Blocks des Zweiblock-Copolymers im Bereich von 1/20 bis 20/1 liegt, und wobei Sequenzcopolymere von Ethylenoxid und Propylenoxid, Sequenzcopolymere mit Urethaneinheiten und Sequenzcopolymere mit Siloxaneinheiten ausgenommen sind.

2. Kosmetische Zusammensetzung nach Anspruch 1, **dadurch gekennzeichnet, dass** das assoziative Polymer, das mindestens einen hydrophilen Teil und mindestens eine C₈₋₄₀-Fettkette aufweist, in dem verwendeten wässrigen Medium löslich ist.

3. Kosmetische Zusammensetzung nach einem der vorhergehenden Ansprüche, **dadurch gekennzeichnet, dass** das oder die linearen Zweiblock-Sequenzcopolymere in dem verwendeten wässrigen Medium löslich sind.

4. Kosmetische Zusammensetzung nach einem der vorhergehenden Ansprüche, **dadurch gekennzeichnet, dass** der hydrophile Block des linearen Zweiblock-Sequenzcopolymers aus anionischen wasserlöslichen Monomeren oder einem Gemisch aus anionischen und nichtionischen wasserlöslichen Monomeren gebildet wird.

5. Kosmetische Zusammensetzung nach Anspruch 4, **dadurch gekennzeichnet, dass** die anionischen wasserlöslichen Monomere unter den Carbonsäuren mit ethylenisch ungesättigter Bindung, 2-Acrylamido-2-methylpropansulfonsäure, Styrolsulfonsäure, Vinylsulfonsäure und Vinylphosphonsäure und den Salzen dieser Säuren ausgewählt sind.

6. Kosmetische Zusammensetzung nach Anspruch 4, **dadurch gekennzeichnet, dass** die nichtionischen wasserlöslichen Monomere unter Acrylamid, N- C₁₋₆-alkylierten Acrylamiden oder N,N-di(C₁₋₃) alkylierten Acrylamiden, Polyethylenglycolacrylat, Polyethylenglycolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen aufweisen, Vinylalkohol, Ethylenoxid, Hydroxyethylacrylat, Hydroxypropylacrylat, Hydroxyethylmethacrylat und Hydroxypropylmethacrylat ausgewählt sind.

7. Kosmetische Zusammensetzung nach einem der vorhergehenden Ansprüche, **dadurch gekennzeichnet, dass** der hydrophobe Block des linearen Zweiblock-Sequenzcopolymers aus in Wasser unlöslichen Monomeren gebildet wird, die unter den vinylaromatischen Monomeren, Dienen und alkylierten Derivaten von Dienen, Chloropren, C₁₋₁₀-Alkyl-acrylaten, C₆₋₁₀-Arylacrylaten, C₁₋₁₀-Aralkylacrylaten, C₁₋₁₀-Alkyl-methacrylaten, C₆₋₁₀-Aryl-methacrylaten, C₁₋₁₀-Aralkyl-methacrylaten, Vinylacetat, Vinylethern der Formel CH₂=CH-O-R und Allylethern der Formel CH₂=CH-CH₂-O-R, worin R eine C₁₋₆-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind.

8. Kosmetische Zusammensetzung nach einem der vorhergehenden Ansprüche, **dadurch gekennzeichnet, dass** der hydrophile Block des linearen Zweiblock-Sequenzcopolymers bis zu 25 Mol-%, vorzugsweise bis zu 10 Mol-% und idealerweise bis zu 5 Mol-% eines oder mehrerer in Wasser unlöslicher Monomere nach Anspruch 7 enthält.

9. Kosmetische Zusammensetzung nach einem der vorhergehenden Ansprüche, **dadurch gekennzeichnet, dass** der hydrophobe Block des linearen Zweiblock-Sequenzcopolymers bis zu 25 Mol-%, vorzugsweise bis zu 10 Mol-% und idealerweise bis zu 5 Mol-% eines oder mehrerer in Wasser löslicher Monomere nach einem der Ansprüche 5 und 6 enthält.

10. Kosmetische Zusammensetzung nach einem der vorhergehenden Ansprüche, **dadurch gekennzeichnet, dass** das oder die linearen Zweiblock-Sequenzcopolymere eine hydrophobe Homopolymer-Sequenz und eine anionische hydrophile Homopolymer-Sequenz enthalten.

11. Kosmetische Zusammensetzung nach Anspruch 10, **dadurch gekennzeichnet, dass** die hydrophobe Homopolymer-Sequenz Polystyrol ist und die anionische hydrophile Homopolymer-Sequenz Poly(natriumacrylat) oder Poly(natriumstyrolsulfonat) ist.

12. Kosmetische Zusammensetzung nach einem der vorhergehenden Ansprüche, **dadurch gekennzeichnet, dass** die Konzentration des assoziativen Polymers im Bereich von 0,01 bis 20 %, vorzugsweise 0,05 bis 10 % und noch bevorzugter im Bereich von 0,1 bis 5 % des Gesamtgewichts der Zusammensetzung liegt.

13. Kosmetische Zusammensetzung nach einem der vorhergehenden Ansprüche, **dadurch gekennzeichnet, dass** die Konzentration des linearen Zweiblock-Sequenzcopolymers im Bereich von 0,001 bis 20 %, vorzugsweise 0,005 bis 10 % und noch bevorzugter im Bereich von 0,01 bis 5 % des Gesamtgewichts der Zusammensetzung liegt.

14. Kosmetische Zusammensetzung nach einem der vorhergehenden Ansprüche, **dadurch gekennzeichnet, dass** das Gewichtsverhältnis assoziative(s) Polymer(e)/Zweiblock-Polymer(e) im Bereich von 0,01 bis 100, vorzugsweise 1 bis 50 und noch bevorzugter 5 bis 30 liegt.

15. Verwendung der Kombination aus
- mindestens einem assoziativen kationischen Polymer, das mindestens einen hydrophilen Teil und mindestens eine C₈₋₄₀-Fettkette aufweist und das unter den Cellulosen und Hydroxyethylcellulosen ausgewählt ist, die quaternisiert sind und Alkyl-, Arylalkyl-, Alkylarylgruppen mit 8 bis 30 Kohlenstoffatomen tragen, und
- mindestens einem anionischen linearen Zweiblock-Sequenzcopolymer, das aus einem hydrophoben Block und einem hydrophilen Block besteht, wobei das Gewichtsverhältnis des hydrophoben Blocks zu dem hydrophilen Blocks des Zweiblock-Copolymers im Bereich von 1/20 bis 20/1 liegt, und wobei Sequenzcopolymere von Ethylenoxid und Propylenoxid, Sequenzcopolymere mit Urethaneinheiten und Sequenzcopolymere mit Siloxaneinheiten ausgenommen sind,
im Bereich der Haarbehandlung.

16. Verwendung der Kombination aus
- mindestens einem assoziativen kationischen Polymer, das mindestens einen hydrophilen Teil und mindestens eine C₈-₄₀-Fettkette aufweist und das unter den Cellulosen und Hydroxyethylcellulosen ausgewählt ist, die quaternisiert sind und Alkyl-, Arylalkyl- und Alkylarylgruppen mit 8 bis 30 Kohlenstoffatomen tragen, und
- mindestens einem anionischen linearen Zweiblock-Sequenzcopolymer, das aus einem hydrophoben Block und einem hydrophilen Block besteht, wobei das Gewichtsverhältnis des hydrophoben Blocks zu dem hydrophilen Blocks des Zweiblock-Copolymers im Bereich von 1/20 bis 20/1 liegt, und wobei Sequenzcopolymere von Ethylenoxid und Propylenoxid, Sequenzcopolymere mit Urethaneinheiten und Sequenzcopolymere mit Siloxaneinheiten ausgenommen sind,
um wässrige kosmetische Zusammensetzungen zu verdicken oder zu gelieren.

17. Verwendung der Kombination nach Anspruch 16, um wässrige Zusammensetzungen für die Haarbehandelung zu verdicken oder zu gelieren.
